## Europäisches Patentamt
## European Patent Office
## Office européen des brevets

(19)

(11) Publication number: **0 122 709**

**A1**

(12)

# EUROPEAN PATENT APPLICATION

(21) Application number: **84301585.0**

(22) Date of filing: **09.03.84**

(51) Int. Cl.³: **A 61 K 9/00**
**A 61 K 31/65, A 61 K 9/24**
**A 61 K 31/565**

(30) Priority: **18.03.83 US 476747**

(43) Date of publication of application:
**24.10.84 Bulletin 84/43**

(84) Designated Contracting States:
**AT BE CH DE FR GB IT LI LU NL SE**

(71) Applicant: **ELI LILLY AND COMPANY**
**307, East McCarty Street**
**Indianapolis Indiana 46285(US)**

(72) Inventor: **Ferguson, Thomas Harry**
**R.R.3 Box 349**
**Greenfield Indiana 46140(US)**

(72) Inventor: **Parrott III, John Calvin**
**222 Cloverdale Drive**
**Council Bluffs Iowa 51501(US)**

(74) Representative: **Crowther, Terence Roger et al,**
**Erl Wood Manor**
**Windlesham Surrey GU20 6PH(GB)**

(54) Improvements in or relating to antibiotic or germicide layered implants.

(57) An improved solid, cylindrical, subcutaneous implant of the type containing a biocompatible inert core having a diameter of from about 2 to about 10 mm. and a biocompatible coating having a thickness of from about 0.2 to about 1 mm., the composition of the coating being from about 5 to about 40 percent by weight of estradiol and from about 95 to about 60 percent by weight of a dimethylpolysiloxane silicone rubber, in which the improvement comprises an effective layer of an antibiotic or germicide on said coating is useful for extending implant retention times.

This invention also includes a method for the administration of estradiol to a ruminant animal, especially a steer, to improve the retention rate of the implant, placing the implant of this invention under the skin of the animal.

EP 0 122 709 A1

Croydon Printing Company Ltd.

## IMPROVEMENTS IN OR RELATING TO
## ANTIBIOTIC OR GERMICIDE LAYERED IMPLANTS

This invention relates to an improvement of nondegradable animal implants, which release a drug contained within the implant at a substantially constant rate. The improvement involved is an antibiotic or germicide layer or coating covering the implant, and which ultimately results in lowered implant loss rates upon implantation.

During implantation, conditions are typically unsanitary, causing infection which often results in the loss of implants. Use of an antibiotic or germicide layer or coating on the implant has been discovered to improve the implant retention, thereby allowing continued controlled release of the drug from the implant, even under unsanitary conditions. In addition, this invention allows for easy application of the implants under unsterile conditions because cleaning of the implant needle, animal, and implanter are no longer needed.

The use of controlled-release implants for drug delivery has been described in the literature and in U.S. Patents 3,279,996; 4,096,239; and 4,191,741. In addition, other instruments for insertion within an animal body, for example, catheters, vaginal suppositories, and sutures, which may contain antibiotics, germicides, or other drugs, have also been described in the the literature.

An improved solid, cylindrical, subcutaneous implant of the type containing a biocompatible inert core having a diameter of from about 2 to about 10 mm. and a biocompatible coating having a thickness of from about 0.2 to about 1 mm., the composition of the coating being from about 5 to about 40 percent by weight of estradiol and from about 95 to about 60 percent by weight of a dimethylpolysiloxane silicone rubber in which the improvement comprises an effective layer of an antibiotic or germicide on the coating has been discovered.

This invention also includes a method for the administration of estradiol to a ruminant animal, especially a steer, by placing the implant of this invention under the skin of the animal. The inserted implant may be removed from the animal prior to slaughter.

Estradiol ($17$-$\beta$-estradiol) is a naturally-occurring estrogen that can be administered to ruminant animals to improve the rate of weight gain of the animals. For optimum results, estradiol should be administered at a substantially constant daily rate.

When an implant as described in U.S. Patent 4,191,741 is placed under the skin of a ruminant animal, an effective amount of estradiol is released from the implant at a substantially constant rate. The estradiol causes the animal to gain weight at a greater than normal rate. At the end of the growing period, the implant, because it remains intact within the animal, can be easily and completely removed, allowing a withdrawal period prior to slaughter.

The present invention, because it helps prevent infection, improves the retention rate of the implants. This improved implant, which contains an antibiotic or germicide layer or coating, remains within the animal until removal at a designated time, rather than being lost earlier due to infection.

The layer or coating may be composed of one or more antibiotics and/or one or more germicides in one or more layers. Typical antibiotics which may be used in this invention include: aminoglycosides, such as gentamicin, kanamycin, neomycin, paromomycin, streptomycin, or tobramycin; ansamycins, such as rifamycin, or rifampin; cephalosporins, such as cephalexin, cephaloridine, cephalothin, cefazolin, cephapirin, cephradine, or cephaloglycin; chloramphenicols; macrolides, such as erythromycin, tylosin, oleandomycin, or spiramycin; penicillins, such as penicillin G & V, phenethicillin, methicillin, oxacillin, cloxacillin, dicloxacillin, floxacillin, nafcillin, ampicillin, amoxicillin, or carbenicillin; sulfonamides; tetracyclines, such as tetracycline, oxytetracycline, chlortetracycline, methacycline, demeclocycline, rolitetracycline, doxycycline, or minocycline; trimethoprim-sulfamethoxazole; polypeptides, such as bacitracin, polymyxins, tyrothricin, or vancomycin; and miscellaneous antibiotics, such as lincomycin, clindamycin, or spectinomycin. The preferred antibiotic, however, is oxytetracycline hydrochloride.

Typical germicides which may be used in this invention include phenols; cresols; resorcinols; substituted phenols; aldehydes; benzoic acid; salicyclic acid; iodine, iodophors, such as betadine; chlorophors, such as hypochlorites; peroxides; such as hydrogen peroxide and zinc peroxide; heavy metals and their salts, such as merbromin, silver nitrate, zinc sulfate; surface-active agents, such as benzalkonium chloride; furan derivatives, such as nitrofurazone; sulfur and thiosulfates; salicylanilides; and carbanilides. Preferred germicides include betadine, iodine, silver nitrate and furan derivatives, such as nitrofurazone.

An effective amount of the antibiotic or germicide is used and is that amount which reduces the implant loss rate. This amount varies with the type of antibiotics or germicides used, the implanting conditions, the method of coating application, the size and surface area of the implant, and the implant retention time desired. For example, the amount of antibiotic can range from about 0.1 mg. per $cm^2$ to about 2.1 mg. per $cm^2$, with a preferred range being from about 0.2 mg. to about 0.8 mg. per $cm^2$. The typical range of the amount of germicide used is exemplified by betadine, which has a range of about 0.5 mg. to about 5.2 mg. per $cm^2$, and by nitrofurazone, which has a range of about 2.0 µg. to about 8.3 µg. per $cm^2$. The preferred ranges of betadine and nitrofurazone are: about 0.5 mg. to about 1.0 mg. per $cm^2$; and about 2.1 µg. to about 4.1 µg. per $cm^2$, respectively. The effective amount of oxytetracycline hydrochloride ranges from about 0.1 mg. to 2.1 mg. per $cm^2$. Preferably the amount of oxytetracycline hydrochloride per implant is from about 0.1 to about 1.0 mg. per $cm^2$ and still more preferably from about 0.14 to about 0.5 mg. per $cm^2$.

The untreated implant is that described in U.S. Patent 4,191,741. Typically, the implant is a cylinder of from about 0.5 to about 6 cm. in length containing an inert, biocompatible core of about 2 to about 10 mm. in diameter with an estradiol-containing coating of dimethylpolysiloxane of about 0.2 to about 1 mm. thickness. The inert, biocompatible core material may be any of a number of substances, but silicone rubber is preferred. The coating is also a silicone rubber, with dimethylpolysiloxane silicone rubbers preferred.

The concentration of estradiol in the coating may vary within the range of from about 5% to about 40%. A preferred range is from about 15% to about 25%. A concentration of 20% of estradiol in the dimethylpolysiloxane has been found to be most preferred.

An effective amount of the estradiol is released from a cylindrical implant having a length of from about 0.5 to about 6 cm. Preferably the cylindrical implant is from about 2 to about 4 cm. in length and still more preferably from about 2.5 to about 3.5 cm. in length. The most preferred length of such a cylindrical implant is about 3 cm.

The diameter of the inert core in such a cylindrical implant is within the range of from about 2 to about 10 mm. A preferred diameter of the inert core is from about 3 to about 5 mm. with the most preferred diameter being about 4.76 mm.

The thickness of the coating applied to the inert core may vary from about 0.2 to about 1 mm., and, preferably from about 0.25 to about 0.5 mm. While it is preferred that the thickness of the coating be uniform, this is not critical.

The rate of release of the estradiol is directly related to the surface area of the implant. The greater the surface area, the greater the rate of release.  An implant that is 3 cm. long and 4.76 mm. in diameter with a 0.25 mm. thick coating has a surface area for release of 4.56 $cm^2$.

The release kinetics of the controlled release implant may be improved by washing the implant in a solvent, removing the peripheral layer of estradiol prior to the addition of the antibiotic or germicide. This results in establishing a zone of crystalline estradiol depletion in the silicone rubber coating and decreases the initial burst release of estradiol.  The overall release rate profile is enhanced because the continuous release of estradiol is more constant.  Wash solvents may include organic solvents such as alcohols, methylene chloride, chloroform, acetone, or dioxane; and aqueous surfactant solutions such as sodium lauryl sulfate, and the like.  Preferred wash solvents include the alcohols, such as methanol or ethanol.  Preferred wash solutions and residence times are 30 minutes in a 95/5 ethanol/methanol solution or 30-40 seconds in a 75/25 methanol/ethanol solution.

The zone of crystalline estradiol depletion can vary in thickness dependent upon the wash solvent used and the length of time in contact with the wash solvent.  Beneficial effects on the estradiol release profile can be obtained from zones of depletion from 2 μm. to 25 μm.  Preferably the zone of crystalline estradiol depletion is between 10 to about 20 μm. in

thickness.  The preferred thickness of the zone of
crystalline estradiol depletion is about 12 µm.

The untreated controlled release implants
are then dusted or coated with the antibiotic or germicide, such as oxytetracycline hydrochloride, by using
a coating pan.  The implants are coated to excess with
the antibiotic or germicide and transferred to a
vibrating bowl or screen and vibrated for approximately
5 to 10 minutes to remove the excess antibiotic or
germicide.  Preferred residence times in the coating
pan and the vibrating bowl or screen are 10 minutes and
5 minutes, respectively.  Preferably, the antibiotic
or germicide used should range in particle size from
about <325 mesh (<45 µm.) to 60 mesh (250 µm.) and still
more preferably from about <325 mesh (<45 µm.) to 200
mesh (75 µm.).  The preferred antibiotic or germicide
particle size is <325 mesh (<45 µm.).  It is preferred
to operate the washing procedure as a continuous batch,
in which the implants are still in a continuous rod.
After being washed the rod is cut into the desired
implant lengths and then the implants are coated or
layered with the antibiotic or germicide.  Alternative
methods of applying the antibiotic or germicidal coating
such as aerosols, emulsions, solutions, electrostatic
spraying, or dry blending are known to those skilled
in the art.

After the implants are coated, they are
usually placed subcutaneously in the posterior median
surface of the animal's ear using an implanter fitted
with a sharpened hollow tube or needle.  Implanters
which can be used are known to those skilled in the art.

A preferred implanting system includes an implanter and cartridge-like container for a plurality of implants. The cartridge is transparent and encloses a plurality of enlongated implants arranged side-to-side, transverse to the central axis of the cartridge. The implants may be moved manually along the central axis of the cartridge toward one end and stopped in alignment parallel with the end of the cartridge and with one implant aligned with a pair of openings in the cartridge, one at each end of the implant. The implanter carries a sharpened tubular needle and a plunger, slidable along an axis aligned with the central axis of the tubular needle. The implanter has an opening into which the cartridge may be fitted and retained. The implanter and cartridge have interfitting surfaces to position the cartridge with its pair of openings lying upon the central axis of the hollow needle and to retain the cartridge in the implanter so that the plunger may be passed repeatedly through the pair of openings in the cartridge and move the implants, one after the other, through the sharpened tubular needle into the animals.

Two modifications which improve its sharpened end can be made to the tubular needle. The first modification, which allows a clean slicing of the ear, is made by sandblasting the flattened surfaces of the needle opening leaving a sharp and pointed tip. The sharpened tip slices the ear to allow insertion of the implant, but the sandblasted, dulled surfaces prevent the ear from being oversliced. Therefore, a small incision is made in the animal's ear by the needle modification.

The second modification is accomplished by placing two projections inside the hollow portion of the needle. These projections prevent the accidental ejection or falling-out of the implants from the implanter. The projections do not obstruct the passage of implants through the needle when the plunger is used; they only prevent accidental movement or loss. An indentation is made on the outside of the hollow portion of the needle, which forms a projection on the inside of the needle.

The effectiveness of the implant of this invention in lowering the loss rate is demonstrated in the following trials:

## Trial 1

One hundred-twenty Hereford steers, weighing approximately 800 pounds and implanted in both ears using 240 COMPUDOSE 200® implants (24 mg. estradiol controlled-release implants, Elanco Products Company, a Division of Eli Lilly and Company), were used to evaluate two dosages of oxytetracycline hydrochloride dusting on estradiol controlled-release implants. The steers were divided into three groups for treatment with untreated control implants; implants with approximately 0.5 mg. of oxytetracycline hydrochloride dusting; and implants with approximately 1.0 mg. of oxytetracycline hydrochloride dusting. The implanting procedure used was as follows: All ears were coated with a manure slurry just prior to implanting and each implant was placed subcutaneously using an implant

X-5967                              -10-

needle, in the middle third of the dorsal side of the ear. All animals were fed a growing ration ad libitum. Implant retention and site infection were recorded on days 7, 15, and 28. On day 1, only implant retention was recorded. The results are shown below:

## Implant Retention

| Treatment | | Cumulative Implants Lost | | | |
|---|---|---|---|---|---|
| Coating oxytetracycline hydrochloride mg. | No. Obs. | Day 1 | Day 7 | Day 15 | Day 28 |
| 0 | 80 | 0 | 6 | 37 | 43 |
| 0.5 | 80 | 0 | 2 | 21 | 27 |
| 1.0 | 80 | 0 | 1 | 16 | 20 |

## Implant Site Infection

| Treatment | Sites Infected | | | | | | | | |
|---|---|---|---|---|---|---|---|---|---|
| | Day 7 | | | Day 15 | | | Day 28 | | |
| | | No. | % | | No. | % | | No. | % |
| Control | (74)[a] | 26 | 35.1 | (43)[a] | 8 | 18.6 | (37)[a] | 5 | 13.5 |
| 0.5 mg. | (78) | 13 | 16.7 | (59) | 7 | 11.9 | (53) | 5 | 5.7 |
| 1.0 mg. | (79) | 4 | 5.1 | (64) | 4 | 6.2 | (60) | 1 | 1.7 |

[a]No. of implants remaining

X-5967

-11-

0122709

## Trial 2

Ninety-six steers, weighing approximately 450 pounds, were implanted in one ear with COMPUDOSE 200® to evaluate the effect of dusting the implants with oxytetracycline hydrochloride powder upon retention in the ear. The implanting procedure used was similar to that described in Trial 1, except that the ears were palpated 1 and 56 days after implanting. Untreated control implants were compared with implants dusted with 1.5 mg. (1.3-1.7 mg. range) per implant of oxytetracycline hydrochloride. The results are as follows:

| Treatment | No. Steers | Implants Retained, Lost, Infected | | | | | |
| --- | --- | --- | --- | --- | --- | --- | --- |
| | | 1st day | | 56th day | | | |
| | | Retained | Inf. | Retained | Lost | Inf. | |
| Oxytetracycline hydrochloride | 48 | 48 | 0 | 43 | 5 | 1 | |
| Control | 48 | 48 | 0 | 34 | 14 | 1 | |

The following trial was conducted as described
in Trial 2.  Trial 3 used 1.5 mg (1.3-1.7 mg. range) per
implant of oxytetracycline hydrochloride.  Steers for
Trial 3 weighed between 500 and 525 pounds:

## Trial 3

### Implants Retained, Lost, Infected

| Treatment | No. Steers | 1st day | | 28th day | | | 56th day | | |
|---|---|---|---|---|---|---|---|---|---|
| | | Retained | Inf. | Retained | Lost | Inf. | Retained | Lost | Inf. |
| Oxytetracycline hydrochloride | 78 | 78 | 0 | 59 | 19 | 5 | 59 | 19 | 2 |
| Control | 80 | 80 | 0 | 32 | 48 | 10 | 31 | 49 | 11 |

X-5967                          -16-

## Trial 4

Following the procedure outlined in Trial 2, 240 Hereford steers were used and divided into three groups: control; oxytetracycline hydrochloride treated implants with from 1.6 to 2.6 mg. per implant (2.2 mg. mean); and TYLAN/Neomycin treated implants. The preparation of TYLAN and Neomycin was applied to the tip of the implant needle, so that approximately 70 mg. per implantation was present on the needle tip prior to implanting. (The preparation is delivered directly to the ear during the implanting process and is not on the implant.) Implant retention and site infection were recorded on days 4 and 26 and are recorded below:

## Trial 4

| | No. | Implants Retained, Lost, Infected | | | | | |
|---|---|---|---|---|---|---|---|
| | | 4th day | | | 26th day | | |
| Treatment | Steers | Retained | Lost | Inf. | Retained | Lost | Inf. |
| Control | 80 | 77 | 3 | 15 | 68 | 12 | 5 |
| TYLAN + Neomycin[1] | 80 | 79 | 1 | 4 | 78 | 2 | 4 |
| oxytetracycline hydrochloride[2] | 80 | 80 | 0 | 2 | 77[3] | 2 | 2 |

---

[1] Preparation applied to needle point.

[2] Oxytetracycline hydrochloride dusted on implant (approximately 2.2 mg/implant).

[3] Two implants lost, one steer died and implant data not covered.

The following trial was conducted according to the procedure of Trial 2, except that the steers, which weighed about 500 pounds, were submerged in a dipping vat, a common infection source, immediately after implanting, rather than coating the ears with a manure slurry. The implants with oxytetracycline hydrochloride were dusted with about 1.5 mg. (1.3-1.7 mg. range) per implant.

## Trial 6

### Implants Retained, Lost, Infected

| Treatment | No. Steers | 1st day | | 28th day | | | 56th day | | |
|---|---|---|---|---|---|---|---|---|---|
| | | Retained | Inf. | Retained | Lost | Inf. | Retained | Lost | Inf. |
| Oxytetracycline hydrochloride | 67 | 67 | 0 | 66 | 1 | 4 | 64 | 3 | 3 |
| Control | 67 | 67 | 0 | 62 | 5 | 10 | 58 | 9 | 9 |

## CLAIMS

1. A solid, cylindrical, subcutaneous implant containing a biocompatible inert core having a diameter of from about 2 to about 10 mm. and a biocompatible coating having a thickness of from about 0.2 to about 1 mm. and said coating comprising from about 5 to about 40 percent by weight of estradiol and from about 95 to about 60 percent by weight of dimethylpolysiloxane silicone rubber and an effective layer of an antibiotic or germicide on said coating.

2. An implant as claimed in claim 1 in which the biocompatible inert core is a silicone rubber.

3. An implant as claimed in claim 1 or 2 in which the diameter of the inert core is from about 3.5 to about 4.5 mm., the thickness of the coating is from about 0.25 to about 0.5 mm., and the concentration of estradiol in the coating is from about 15 to about 25 percent.

4. An implant as claimed in any one of claims 1 to 3 in which the concentration of estradiol in the coating is about 20 percent.

5. An implant as claimed in claim 4 in which the effective layer is formed with antibiotic.

6. An implant as claimed in claim 5 wherein the amount of antibiotic used is from about 0.1 mg. to about 2.1 mg. per $cm^2$.

7. An implant as claimed in claim 6 in which the antibiotic is oxytetracycline hydrochloride.

8. An implant as claimed in claim 4 in which the effective layer is formed with a germicide.

9. An implant as claimed in claim 8 in which the germicide is betadine, iodine, nitrofurazone, or silver nitrate.

10. An implant as claimed in claim 9 in which the amount of betadine is from about 0.5 mg. to about 5.2 mg. per $cm^2$.

11. An implant as claimed in claim 9 in which the amount of nitrofurazone is from about 2.0 µg. to about 8.3 µg. per $cm^2$.

12. A method for improving the rate of weight gain of ruminant animals which comprises the subcutaneous implantation of an implant according to any one of claims 1 to 11.

0122709

**European Patent Office**

**PARTIAL EUROPEAN SEARCH REPORT**
which under Rule 45 of the European Patent Convention
shall be considered, for the purposes of subsequent
proceedings, as the European search report

Application number

| | DOCUMENTS CONSIDERED TO BE RELEVANT | | EP 84301585.0 |

| Category | Citation of document with indication, where appropriate, of relevant passages | Relevant to claim | CLASSIFICATION OF THE APPLICATION (Int. Cl. ³) |
|---|---|---|---|
| D,Y | US - A - 4 191 741 (HUDSON WAGNER) <br> * Claims 1-5 * | 1 | A 61 K 9/00 <br> A 61 K 9/24 |
| A | * Claims 1-5 * | 2-4 | A 61 K 31/565 <br> A 61 K 31/65 |
| Y | US - A - 3 424 839 (MONTANDRAUD) <br> * Claims 1,2 * | 1 | |
| A | * Claims 1,2 * | 5 | |
| A | DE - A1 - 2 902 414 (HOECHST AG) <br> * Claims 1,6,8,9,11,16; page 6, lines 3-5; page 9, lines 23-26 * | 1,4,5 | |
| A | DE - A - 2 259 070 (THE UPJOHN CO) <br> * Claim 1; page 6, line 17 - page 7, line 15; page 12, lines 9-14 * | 1,2,5, 8 | TECHNICAL FIELDS SEARCHED (Int. Cl. ³) <br> A 61 K 9/00 <br> A 61 K 31/00 <br> A 23 K 1/00 |

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 1-11
Claims searched incompletely: —
Claims not searched: 12
Reason for the limitation of the search:

Method for treatment of the human or animal body by therapy (Article 52(4) EPC)

| Place of search | Date of completion of the search | Examiner |
|---|---|---|
| VIENNA | 16-08-1984 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82'

European Patent
Office

**PARTIAL EUROPEAN SEARCH REPORT**

EP 84301585.0

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| A | GB - A - 2 093 348 (LEO PHARMACEU-TICAL PRODUCTS)<br><br>* Claim 1 *<br><br>-- | 1,5 | |
| A | US - A - 4 333 919 (KLEBER,SIMPSON)<br><br>* Claims 1,2,6 *<br><br>-- | 1,5 | |
| A | GB - A - 2 059 765 (ELI LILLY AND COMPANY)<br><br>* Claims 1-3 *<br><br>-- | 1,5 | |
| A | GB - A - 2 059 764 (ELI LILLY AND COMPANY)<br><br>* Claims 1,3,4,6,10,12-16 *<br><br>-- | 1,5,7,8 | TECHNICAL FIELDS SEARCHED (Int. Cl.³) |
| A | US - A - 3 472 934 (DRAIN)<br><br>* Abstract; claim 1 *<br><br>---- | 1,5,7,8 | |

EPO Form 1505.3   06.78